# EUROPEAN PATENT APPLICATION

(11) **EP 4 180 452 A1**
(43) Date of publication of application: **17.05.2023**
(21) Application number: 21838782.7
(22) Date of filing: 09.07.2021
(51) Int. Cl.: C07K 14/605, C07K 14/00, C12N 15/62, A61K 38/00, A61P 3/00

(54) **FUSION PROTEIN INCLUDING GLUCAGON-LIKE PEPTIDE-1 AND INTERLEUKIN-1 RECEPTOR ANTAGONIST AND USE THEREOF**

(30) Priority: 10.07.2020 KR 20200085355
(71) Applicant: GI Innovation, Inc., Seoul 05855 (KR)
(72) Inventor: JANG, Myung Ho, Seoul 05849 (KR); OH, Young Min, Yongin-si Gyeonggi-do 16847 (KR); OH, Nahyun, Seongnam-si Gyeonggi-do 13477 (KR)
(74) Representative: HGF
(86) International application number: PCT/KR2021/008824
(87) International publication number: WO 2022/010319

(57) **Abstract**

The present invention relates to a fusion protein dimer comprising glucagon-like peptide-1 (GLP-1) and an interleukin-1 receptor antagonist (IL-1Ra), and a pharmaceutical composition for treating a metabolic disease using the same. It was confirmed that the fusion protein dimer significantly acts as a GLP-1 hormone and efficiently binds to an IL-1 receptor, and thus has an excellent inhibitory effect on the signaling of IL-1β caused by inflammasomes. Therefore, the fusion protein dimer according to the present invention has the combined efficacy of GLP-1 and an IL-1 receptor antagonist and can be effectively used for treatment and prevention of metabolic diseases such as diabetes, obesity, and hyperlipidemia, and thus has a high possibility of being industrially used.

## Description

### Technical Field

The present invention relates to a fusion protein dimer comprising glucagon-like peptide-1 (GLP-1) and an interleukin-1 receptor antagonist (II,-1Ra); and a composition for treating a metabolic disease using the same.

### Background Art

In modern society, the obese population is rapidly increasing due to various causes such as excessive nutrition intake resulting from the increase in processed food and eating-out and the decrease in the physical activity due to automation, and accordingly, metabolic diseases such as hypertension, diabetes, arteriosclerosis, hyperlipidemia, insulin resistance, and dyslipidemia are increasing.

The metabolic disease is a generic term for diseases caused by metabolic disorders in the body, and since the causes of the disease are diverse and act in a complicated manner, it is difficult to develop a clear therapeutic method.

Meanwhile, glucagon-like peptide-1 (GLP-1) is an incretin hormone secreted from L cells in the intestinal tract by stimuli such as food, and is known to be associated with a therapeutic effect on diabetes, obesity, heart diseases, cerebrovascular diseases, neuronal inflammation, arteriosclerosis, and the like. In this regard, liraglutide, which is a GLP-1 analogue approved as an anti-obesity drug, has been approved as an adjunct to a reduced-calorie diet and an exercise for weight control. This drug has been approved for use in obese or overweight adults with obesity, hypertension, type 2 diabetes, or dyslipidemia. However, there are still issues of safety and side effects.

An interleukin-1 receptor antagonist (II,-1Ra) is a substance that inhibits IL-1, which is an inflammatory cytokine present in the two forms of IL-1α and IL-1β, and competitively inhibits the binding of IL-1 to an IL-1 receptor to inhibit the effects of IL-1. Meanwhile, IL-1β, which is an important mediator in inflammatory responses, is produced by inflammasomes, and recent studies have found that various metabolic diseases are associated with inflammasomes (Dominic De Nardo et. al., Trends Immunol., 32(8):373-379, 2011). Therefore, studies to treat various diseases such as metabolic diseases associated with inflammasomes and IL-1β, which is a metabolite thereof, by inhibiting inflammasomes and IL-1β are being actively conducted (Charles A Dinarello et. al., Nat Rev Drug Discov., 11(8):633-652, 2012).

### Detailed Description of Invention

### Technical Problem

Accordingly, the present inventors have studied to develop a fusion protein having novel combination for effectively treating and preventing a metabolic disease. As a result, the present inventors have confirmed that a fusion protein dimer comprising GLP-1 and an IL-1 receptor antagonist (IL-1Ra) acts as a GLP-1 hormone and efficiently binds to an IL-1 receptor to inhibit the signal of IL-1, which is an inflammatory cytokine. Based on the result, the present inventors have confirmed that the fusion protein dimer can be usefully used as a therapeutic agent for metabolic diseases such as diabetes and obesity, thereby completing the present invention.

### Solution to Problem

In order to achieve the above object, in an aspect of the present invention, there is provided a fusion protein comprising GLP-1 or a variant thereof and an interleukin-1 receptor antagonist (IL-1 receptor antagonist) or a fragment thereof; and a dimer thereof.

In another aspect of the present invention, there is provided a polynucleotide encoding the fusion protein, an expression vector including the polynucleotide, and a transformed cell into which the expression vector has been introduced.

In yet another aspect of the present invention, there is provided a pharmaceutical composition for preventing or treating a metabolic disease comprising the fusion protein dimer.

In still another aspect of the present invention, there is provided a use of the fusion protein dimer for the manufacture of a medicament for treatment or prevention of a metabolic disease.

In yet still another aspect of the present invention, there is provided a use of the fusion protein dimer for treatment or prevention of a metabolic disease.

In yet still another aspect of the present invention, there is provided a method for preventing or treating a metabolic disease, the method including administering the fusion protein dimer to a subject.

### Effects of Invention

The fusion protein dimer comprising GLP-1 and an IL-1 receptor antagonist (IL-1Ra), according to the present invention can significantly act as a GLP-1 hormone, which is known to promote insulin secretion according to blood glucose concentration and to have an anti-obesity effect. In addition, the fusion protein dimer binds to an IL-1 receptor (IL-1R) and has an excellent inhibitory effect on the signaling of IL-1β produced by inflammasomes. Therefore, in a functional aspect, the fusion protein dimer has the combined efficacy of GLP-1 and an IL-1 receptor antagonist and, thus, can be effectively used for the prevention and treatment of metabolic diseases such as diabetes, obesity, and hyperlipidemia.

### Brief Description of Drawings

FIG. 1 illustrates the structure of GI-210B 1 (GLP-1-IgG4 Fc-IL-1Ra), which is a fusion protein dimer according to the present invention.
FIG. 2 is a picture in which the prepared GI-210B1 was confirmed by SDS-PAGE.
FIG. 3 is a picture in which the prepared GI-210B1 was confirmed by western blot.
FIG. 4 is a graph showing the results obtained by measuring binding capacity of GI-210B1 to interleukin-1 receptor alpha (IL-1Rα).
FIG. 5a illustrates an experimental method for confirming the efficacy of GI-210B 1 as an IL-1 receptor antagonist (IL-1Ra).
FIG. 5b is a graph showing the experimental results obtained by confirming the efficacy of GI-210B 1 as an IL-1 receptor antagonist (II,-1Ra).
FIG. 6 is a graph showing the results obtained by confirming the efficacy of GI-210B 1 as a GLP-1 hormone.
FIG. 7 is pictures and graphs showing the results obtained by confirming the ability of GL210B1 to inhibit fat accumulation in hepatocytes caused by palmitate and oleic acid.

### Best Mode for Carrying out the Invention

### Fusion protein comprising GLP-1 and IL-1 receptor antagonist (IL-1Ra)

In an aspect of the present invention, there is provided a fusion protein comprising GLP-1 or a variant thereof; and an interleukin-1 receptor antagonist (IL-1 receptor antagonist) or a fragment thereof.

As used herein, the term "glucagon-like peptide-1 (GLP-1)" or "glucagon-like peptide-1" refers to an incretin derived from a gene transcription product of proglucagon as a prohormone, which is a hormone secreted from the intestinal L cells when stimulated by nutrients or blood glucose concentration in the intestine.

GLP-1 consists of 30 amino acids, and the amino acid sequence of the GLP-1 is known to be 100% identical in all mammals. It is known that glucagon is produced in pancreatic islet α cells by a post-transcriptional process from proglucagon, while GLP-1 is produced in ileal and colonic L-cells. Specifically, GLP-1 may have the sequence of SEQ ID NO: 1, but is not limited thereto.

In the present specification, GLP-1 or a variant thereof may be collectively referred to as the term "GLP-1". The GLP-1 and the GLP-1 variant specifically bind to, for example, a GLP-1 receptor (GLP-1R, glucagon-like peptide-1 receptor). This specific binding may be identified by methods known to those skilled in the art.

As used herein, the term "GLP-1 variant" refers to a form in which some amino acids of the full-length GLP-1 are substituted, deleted, added, and/or inserted. That is, a GLP-1 variant may have an amino acid sequence different from wild-type GLP-1. However, the GLP-1 variant may have an activity equivalent to or similar to wild-type GLP-1. Here, "GLP-1 activity" may refer to, for example, specific binding to a GLP-1 receptor, and this specific binding may be measured by methods known to those skilled in the art.

Specifically, a GLP-1 variant may be obtained by deleting, modifying, substituting, and/or adding some amino acids of wild-type GLP-1, and may be prepared by a method known to those skilled in the art. The GLP-1 variant may contain at least 5 amino acids, at least 10 amino acids, at least 15 amino acids, or at least 20 amino acids in the sequence of wild-type GLP-1.

Specifically, the GLP-1 variant may be obtained by substituting or deleting some amino acids of wild-type GLP-1. In an embodiment, the GLP-1 variant obtained by the substitution and deletion of amino acids may be obtained by substituting 2^{nd} and 30^{th} amino acids in the amino acid sequence of SEQ ID NO: 1 with other amino acids and adding one amino acid to the C-terminus.

In addition, the GLP-1 variant may be obtained by substituting and adding some amino acids of wild-type GLP-1. In an embodiment, the GLP-1 variant obtained by the substitution and addition of amino acids may be obtained by substituting 2^{nd}, 10^{th}, 12^{th}, 13^{th}, 14^{th}, 16^{th}, 17^{th}, 19^{th}, 20^{th}, 21^{st}, 24^{th}, 27^{th}, 28^{th}, and 30^{th} amino acids in the amino acid sequence of SEQ ID NO: 1 with other amino acids and adding nine amino acids to the C-terminus. Further, the GLP-1 variant may be obtained by substituting 2^{nd} and 30^{th} amino acids in the amino acid sequence of SEQ ID NO: 1 with other amino acids and adding one amino acid to the C-terminus.

Here, the "amino acid" introduced by substitution and/or addition may be any one selected from the group consisting of glycine (G), leucine (L), lysine (K), glutamine (Q), methionine (M), glutamic acid (E), valine (V), arginine (R), asparagine (N), proline (P), serine (S), and alanine (A)

In an embodiment, the GLP-1 variant may be a GLP-1 variant in which A2G and R30G substitutions occur in the amino acid sequence of SEQ ID NO: 1 and one amino acid G is added to the C-terminus. In addition, the GLP-1 variant may be a GLP-1 variant in which A2G, V10L, S12K, Y13Q, L14M, G16E, Q17E, A19V, K20R, E21L, A24E, V27K, K28N, and R30G substitutions occur in the amino acid sequence of SEQ ID NO: 1 and nine amino acids PSSGAPPPS (SEQ ID NO: 21) are added to the C-terminus.

Specifically, the GLP-1 variant may have the amino acid sequence of any one of SEQ ID NOs: 2 to 4.

In addition, the GLP-1 variant may be characterized in that the duration of action in the body is extended. Wild-type GLP-1 has many difficulties in being developed as a drug, including being cleaved and inactivated at a very high speed by a dipeptidyl peptidase-4 (DPP-4) enzyme in the body. Accordingly, the GLP-1 variant is a variant in which cleavage by a DPP-4 enzyme is reduced by causing a variation in wild-type GLP-1, and may be characterized by having a biological half-life significantly longer than that of wild-type GLP-1 (1 to 2 minutes).

Meanwhile, the wild-type GLP-1 is processed in the body such that the first six amino acids are cleaved from the molecule. Therefore, according to the general practice in the art, the amino-terminus of GLP-1 is designated as number 7 and the carboxy-terminus thereof is designated as number 37. GLP-1 (1-37), which is a form having no insulin secretion function, can be processed into the active form of GLP-1 (7-37) according to the aforementioned processing and can be further modified in the body such that a glycine residue at the C-terminus is removed and substituted with an amide group to become a GLP-1 (7-36) amide (amino acid sequence of SEQ ID NO: 1).

"GLP-1" or "glucagon-like peptide-1" refers to, unless otherwise stated, any wild-type GLP-1 obtained from any vertebrate sources, including mammals, for example, primates (for example, humans) and rodents (for example, mice and rats). The GLP-1 may be obtained from animal cells, but also includes those obtained from recombinant cells capable of producing GLP-1. In addition, the GLP-1 may be wild-type GLP-1 or a variant thereof.

As used herein, the term "interleukin-1 receptor antagonist (IL-1 receptor antagonist)" or "IL-1Ra" refers to an antagonist that inhibits IL-1 present in the two forms of IL-1α and IL-1β, and II,-1Ra is structurally similar to IL-1α and IL-1β and thus competitively binds to an IL-1 receptor (IL-1R) to inhibit IL-1 that is an inflammatory cytokine. Therefore, II,-1Ra is recognized to play an important role in preventing diseases caused by IL-1. Meanwhile, II,-1Ra is known to have a pathophysiologically important action in type 2 diabetes and atherosclerosis. Specifically, the II,-1Ra may have the amino acid sequence of SEQ ID NO: 8, but is not limited thereto.

In the present specification, an IL-1 receptor antagonist (IL-1Ra) or a fragment thereof may be collectively referred to as the term "IL-1Ra". The II,-1Ra and the II,-1Ra fragment specifically bind to, for example, an IL-1 receptor (IL-1R, interleukin-1 receptor). This specific binding can be confirmed by a method known to those skilled in the art.

As used herein, the term "II,-1Ra fragment" refers to a cleaved form of the IL-1Ra, and may comprise a fragment in which a portion of the N-terminus or C-terminus of the full-length II,-1Ra is truncated. In addition, the II,-1Ra fragment can be used in the same context as the "II,-1Ra variant", and refers to a form in which some amino acids of the full-length II,-1Ra are substituted, deleted, added, and/or inserted. That is, an II,-1Ra variant may have an amino acid sequence different from wild-type IL-1Ra. However, the II,-1Ra variant may have an activity equivalent to or similar to wild-type IL-1Ra. Here, "II,-1Ra activity" may refer to, for example, specific binding to an IL-1 receptor, and this specific binding may be measured by methods known to those skilled in the art.

Specifically, an II,-1Ra variant may be obtained by deleting, modifying, substituting, and/or adding some amino acids of wild-type IL-1Ra, and may be prepared by a method known to those skilled in the art. The II,-1Ra variant may contain at least 5 amino acids, at least 10 amino acids, at least 15 amino acids, at least 20 amino acids, at least 30 amino acids, at least 40 amino acids, at least 50 amino acids, at least 60 amino acids, at least 70 amino acids, at least 80 amino acids, at least 90 amino acids, or at least 100 amino acids of a wild-type IL-1Ra.

"IL-1Ra" or "IL-1 receptor antagonist (II,-1Ra)" refers to, unless otherwise stated, any wild-type II,-1Ra obtained from any vertebrate sources, including mammals, for example, primates (for example, humans) and rodents (for example, mice and rats). The II,-1Ra may be obtained from animal cells, but also includes those obtained from recombinant cells capable of producing IL-1Ra. In addition, the II,-1Ra may be a wild-type II,-1Ra or a fragment thereof.

The fusion protein may comprise an immunoglobulin Fc region. Here, an Fc domain of the immunoglobulin refers to a protein which includes a heavy chain constant region 2 (CH2) and a heavy chain constant region 3 (CH3) of the immunoglobulin, and does not include variable regions of heavy and light chains and a light chain constant region 1 (CL1) of the immunoglobulin. The immunoglobulin may be IgG, IgA, IgE, IgD, or IgM, and preferably IgG4.

In addition, the Fc domain of an immunoglobulin may be an Fc domain variant as well as a wild-type Fc domain. In addition, as used herein, the term "Fc domain variant", may refers to a form which is different from the wild-type Fc domain in terms of a glycosylation pattern, has a high glycosylation as compared with the wild-type Fc domain, or has a low glycosylation as compared with the wild-type Fc domain, or a deglycosylated form. In addition, an aglycosylated Fc domain is included therein. The Fc domain or a variant thereof may be adapted to have an adjusted number of sialic acids, fucosylations, or glycosylations through culture conditions or genetic manipulation of a host.

In addition, glycosylation of the Fc domain of an immunoglobulin may be modified by conventional methods, such as a chemical method, enzymatic method, and genetic engineering method using microorganisms. In addition, the Fc domain variant may be in a mixed form of respective Fc regions of immunoglobulin IgG, IgA, IgE, IgD, or IgM. In addition, the Fc domain variant may be in a form in which some amino acids of the Fc domain are substituted with other amino acids. In an embodiment, a Fc domain variant may have the amino acid sequence of SEQ ID NO: 6.

Specifically, the fusion protein may consist of structural formula (I) or (II).
N'-X-[linker (1)]n-Fc region fragment or variant thereof-[linker (2)]m-Y-C' (I)
N'-Y-[linker (1)]n-Fc region fragment or variant thereof-[linker (2)]m-X-C' (II)
Here, in the structural formulae (I) and (II),
N' is the N-terminus of the fusion protein,
C' is the C-terminus of the fusion protein,
X is GLP-1 or a variant thereof,
Y is an IL-1 receptor antagonist or a fragment thereof,
the linker (1) and the linker (2) are peptide linkers, and
n and m are each independently 0 or 1.

Here, the GLP-1 or a variant thereof and the IL-1 receptor antagonist or a fragment thereof are as described above.

Here, the peptide linker (1) may consist of 5 to 80 contiguous amino acids, 10 to 70 contiguous amino acids, 15 to 60 contiguous amino acids, 20 to 50 contiguous amino acids, or 25 to 40 amino acids. In an embodiment, the peptide linker (1) may consist of 30 amino acids. In addition, the peptide linker (1) may contain at least one cysteine. Specifically, the peptide linker (1) may contain one, two, or three cysteines. In addition, the peptide linker (1) may be derived from a hinge of an immunoglobulin. In an embodiment, the peptide linker (1) may be a peptide linker consisting of the amino acid sequence of SEQ ID NO: 5.

The peptide linker (2) may consist of 1 to 50 contiguous amino acids, 3 to 30 contiguous amino acids, or 5 to 20 amino acids. The peptide linker (2) may include (G₄S)n (here, n is an integer of 1 to 10), and may further include (G)n. Here, n in (G₄S)n and (G)n may each be independently 1, 2, 3, 4, 5, 6, 7, 8, 9, or 10. In an embodiment, the peptide linker (2) may be a peptide linker consisting of the amino acid sequence of SEQ ID NO: 7.

The amino acid sequences of each of the regions constituting the fusion protein is as shown in Table 1 below. In addition, in an embodiment, GI-210B1 (GLP-1-IgG4 Fc-II,-1Ra) may have the amino acid sequence of SEQ ID NO: 10.

**[Table 1]**

| Classification | Amino acid sequence | SEQ ID NO: |
|---|---|---|
| hGLP-1 | HAEGTFTSDV SSYLEGQAAK EFIAWLVKGR | 1 |
| GLP-1 variant-1 | HGEGTFTSDV SSYLEEQAAK EFIAWLVKGG G | 2 |
| GLP-1 variant-2 | HGEGTFTSDL SKQMEEEAVR LFIEWLKNGG PSSGAPPPS | 3 |
| GLP-1 variant-3 | HAEGTFTSDV SSYLEGQAAK EFIAWLVRGR G | 4 |
| First linker | GGGGSGGGGS GGGGSGGAES KYGPPCPPCP | 5 |
| hIgG4Fc | APEAAGGPSVFLFPPKPKDQLMISRTPEVTCVV VDVSQEDPEVQFNWYVDGVEVHNAKTKPREE QFNSTYRVVSVLTVLHQDWLNGKEYKCKVSN KGLPSSIEKTISKAKGQPREPQVYTLPPSQEEMT KNQVSLTCLVKGFYPSDIAVEWESNGQPENNY KTTPPVLDSDGSFFLYSRLTVDKSRWQEGNVFS CSVLHEALHNHYTQKSLSLSLG | 6 |
| Second linker | GGGGSGGGGSGGGGSGG | 7 |
| IL-1Ra | RPSGRKSSKMQAFRIWDVNQKTFYLRNNQLVA GYLQGPNVNLEEKIDVVPIEPHALFLGIHGGKM CLSCVKSGDETRLQLEAVNITDLSENRKQDKRF AFIRSDSGPTTSFESAACPGWFLCTAMEADQPV SLTNMPDEGVMVTKFYFQEDE | 8 |
| GI-210B1 (GLP-1-IgG4 Fc-IL-1Ra) | HGEGTFTSDV SSYLEEQAAK EFIAWLVKGG G GGGGSGGGGSGGGGSGGAESKYGPPCPPCP APEAAGGPSVFLFPPKPKDQLMISRTPEVTCVV VDVSQEDPEVQFNWYVDGVEVHNAKTKPREE QFNSTYRVVSVLTVLHQDWLNGKEYKCKVSN KGLPSSIEKTISKAKGQPREPQVYTLPPSQEEMT | 10 |
| | KNQVSLTCLVKGFYPSDIAVEWESNGQPENNY KTTPPVLDSDGSFFLYSRLTVDKSRWQEGNVFS CSVLHEALHNHYTQKSLSLSLG GGGGSGGGGSGGGGSGG RPSGRKSSKMQAFRIWDVNQKTFYLRNNQLVA GYLQGPNVNLEEKIDVVPIEPHALFLGIHGGKM CLSCVKSGDETRLQLEAVNITDLSENRKQDKRF AFIRSDSGPTTSFESAACPGWFLCTAMEADQPV SLTNMPDEGVMVTKFYFQEDE | |

### Fusion protein dimer

In another aspect of the present invention, there is provided a fusion protein dimer in which the two fusion proteins are bound. Here, the fusion proteins may be bound by a cysteine included in a linker that links the GLP-1 or a variant thereof and the immunoglobulin Fc region. Here, the linker may include a hinge region of an immunoglobulin.

### Polynucleotide encoding fusion protein

In yet another aspect of the present invention, there is provided a polynucleotide encoding a fusion protein comprising GLP-1 or a variant thereof and an IL-1 receptor antagonist (IL-1Ra) or a fragment thereof. Here, in an embodiment, the polynucleotide may have SEQ ID NO: 18 or SEQ ID NO: 20.

The GLP-1 or a variant thereof and the IL-1 receptor antagonist or a fragment thereof are as described above.

The polynucleotide may further contain a signal sequence or a leader sequence. As used herein, the term "signal sequence" refers to a nucleic acid encoding a signal peptide that directs the secretion of a target protein. The signal peptide is translated and then cleaved in a host cell. Specifically, the signal sequence according to the present invention is a nucleotide encoding an amino acid sequence that initiates migration of a protein across the endoplasmic reticulum (ER) membrane.

Signal sequences are well known in the art for their characteristics. such signal sequences typically contain 16 to 30 amino acid residues, and may contain more or fewer amino acid residues than such amino acid residues. A typical signal peptide is composed of three regions, that is, a basic N-terminal region, a central hydrophobic region, and a more polar C-terminal region. The central hydrophobic region contains 4 to 12 hydrophobic residues that cause the signal sequence to be immobilized during migration of an immature polypeptide through the membrane lipid bilayer.

After initiation, signal sequence is cleaved in the lumen of the ER by cellular enzymes commonly known as signal peptidases. Here, the signal sequence may be a secretory signal sequence of tPa (tissue plasminogen activation), HSV gDs (a signal sequence of herpes simplex virus glycoprotein D), an IgG signal sequence, or a growth hormone. Preferably, a secretory signal sequence used in higher eukaryotic cells including mammals and the like may be used.

Signal sequences useful in the present invention include a light chain signal sequence of an antibody, such as a 14.18 antibody (Gillies et al., J. Immunol. Meth 1989. 125: 191-202), a heavy chain signal sequence of an antibody, such as a heavy chain signal sequence of an MOPC141 antibody (Sakano et al., Nature, 1980. 286: 676-683), and other signal sequences known in the art (see, for example, Watson et al., Nucleic Acid Research, 1984, 12: 5145-5164). In an embodiment, a signal sequence consisting of the amino acids of SEQ ID NO: 9 was used as a signal sequence.

### Vector carrying polynucleotide

In still another aspect of the present invention, there is provided an expression vector including the polynucleotide encoding the fusion protein comprising GLP-1 or a variant thereof and the IL-1 receptor antagonist or a fragment thereof. Here, the polynucleotide may have the sequence of SEQ ID NO: 18.

As used herein, the term "vector" is intended to be introduced into a host cell and to be capable of being recombined with a host cell genome and inserted therein. Alternatively, the vector is an episome and is understood to be a nucleic acid unit containing a nucleotide sequence that can be autonomously replicated. Here, the vector may be operably linked to an appropriate promoter so that the polynucleotide can be expressed in a host cell. The vectors include linear nucleic acids, plasmids, phagemids, cosmids, RNA vectors, viral vectors, minichromosomes, and analogs thereof. Examples of the viral vector include, but are not limited to, retroviruses, adenoviruses, and adeno-associated viruses. In addition, the plasmid may include a selectable marker such as an antibiotic-resistant gene, and host cells harboring the plasmid may be cultured under selective conditions.

As used herein, the term "gene expression" or "expression" of the target protein is understood to mean transcription of DNA sequences, translation of mRNA transcripts, and secretion of fusion protein products or fragments thereof. A useful expression vector may be RcCMV (Invitrogen, Carlsbad) or a variant thereof. The expression vector may further contain a human cytomegalovirus (CMV) promoter for promoting continuous transcription of a target gene in mammalian cells, and a bovine growth hormone polyadenylation signal sequence for increasing the steady-state level of RNA after transcription.

### Transformed cell expressing fusion protein

In yet still another aspect of the present invention, there is provided a transformed cell into which the expression vector including the polynucleotide encoding the fusion protein comprising GLP-1 or a variant thereof and an IL-1 receptor antagonist or a fragment thereof has been introduced.

As used herein, the term "transformed cell" refers to a prokaryotic cell or an eukaryotic cell into which a recombinant expression vector can be introduced. The transformed cell can be prepared by introducing a vector into a host cell to transform the host cell. In addition, the fusion protein according to the present invention can be produced by expressing the polynucleotide included in the vector.

The transformation can be performed by various methods. The method is not particularly limited as long as the fusion protein according to the present invention can be produced thereby. Specifically, as the transformation method, CaCl₂ precipitation, Hanahan method whose efficiency has been increased by using a reducing agent such as dimethyl sulfoxide (DMSO) in the CaCl₂ precipitation, electroporation, calcium phosphate precipitation, protoplast fusion, agitation using silicon carbide fiber, Agrobacterium-mediated transformation, transformation using PEG, dextran sulfate, lipofectamine, a dry/inhibition-mediated transformation, and the like can be used. In addition, a target can be delivered into a cell using infection as a means and using viral particles. In addition, the vector is introduced into the host cell, gene bombardment or the like may be used.

In addition, the host cell used for the production of the transformed cell is also not particularly limited as long as the host cell can produce the fusion protein according to the present invention. Specifically, the host cell may include prokaryotic cells, eukaryotic cells, and cells of mammals, plants, insects, fungi, or any cellular origin. As an example of the prokaryotic cells, *Escherichia coli* may be used. In addition, as an example of the eukaryotic cells, yeast may be used. In addition, for the mammalian cells, CHO cells, F2N cells, COS cells, BHK cells, Bowes melanoma cells, HeLa cells, 911 cells, AT1080 cells, A549 cells, SP2/0 cells, human lymphoblastoids, NSO cells, HT-1080 cells, PERC.6 cells, HEK 293 cells, HEK293T cells, or the like can be used. However, the mammalian cells are not limited thereto, and any cells which are known to those skilled in the art to be usable as a mammalian host cells may all be used.

### Method for preparing fusion protein

In yet still another aspect of the present invention, there is provided a method for preparing a fusion protein dimer comprising GLP-1 or a variant thereof and an IL-1 receptor antagonist or a fragment thereof.

The method for preparing a fusion protein may include (i) culturing the transformed cell; and (ii) collecting a fusion protein dimer comprising GLP-1 or a variant thereof and an IL-1 receptor antagonist or a fragment thereof.

As used herein, the term "culturing" refers to a method for artificially growing microorganisms under appropriately controlled environmental conditions.

Culturing the transformed cell may be carried out using methods well known in the art. Specifically, the culturing is not particularly limited as long as the fusion protein according to the present invention can be produced by expression. Specifically, the culture may be carried out in a batch process, or carried out continuously in a fed batch or repeated fed batch process.

In addition, collecting the fusion protein from the cultured matter may be performed by methods known in the art. Specifically, the collection method is not particularly limited as long as the produced fusion protein according to the present invention can be collected. Preferably, the collection method may be a method such as centrifugation, filtration, extraction, spraying, drying, evaporation, precipitation, crystallization, electrophoresis, fractional dissolution (for example, ammonium sulfate precipitation), or chromatography (for example, ion exchange, affinity, hydrophobicity, and size exclusion).

### Use of fusion protein dimer

In yet still another aspect of the present invention, there is provided a pharmaceutical composition for preventing or treating a metabolic disease comprising the fusion protein dimer. Here, a use of the pharmaceutical composition may be for preventing or treating a metabolic disease.

As described above, the fusion protein may comprise GLP-1 or a variant thereof and an IL-1 receptor antagonist or a fragment thereof.

As used herein, the term "metabolic disease" is a generic term for diseases caused by metabolic disorders in the body, and the metabolic disease may be selected from the group consisting of diabetes, obesity, hyperlipidemia, hypertension, arteriosclerosis, and hyperinsulinemia, but is not limited thereto.

A preferred dosage of the pharmaceutical composition varies depending on the patient's conditions and body weight, the severity of a disease, a form of drug, and the route and duration of administration, and may be appropriately selected by those skilled in the art. In the pharmaceutical composition for treating or preventing a metabolic disease according to the present invention, the fusion protein may be contained in any amount (effective amount) depending on application, a dosage form, a blending purpose, or the like as long as the fusion protein dimer can exhibit an activity of treating a metabolic disease or, in particular, a therapeutic effect on diseases such as diabetes and obesity, and a conventional effective amount thereof would be determined within the range of 0.001 wt% to 20.0 wt%, based on the total weight of the composition. Here, the term "effective amount" refers to an amount of an active ingredient capable of inducing the effect of ameliorating or treating a disease and, in particular, the effect of ameliorating or treating metabolic diseases such as diabetes and obesity. Such an effective amount can be experimentally determined within the scope of common knowledge of of those skilled in the art.

As used herein, the term "treatment" may be used to mean both therapeutic and prophylactic treatment. Here, prophylaxis may be used to mean that a pathological condition or disease of an individual is alleviated or mitigated. In an embodiment, the term "treatment" includes both application or any form of administration for treating a disease in a mammal, including a human. In addition, the term includes inhibiting or slowing down a disease or disease progression; and includes meanings of restoring or repairing impaired or lost function so that a disease is partially or completely alleviated; stimulating inefficient processes; or alleviating a serious disease.

Pharmacokinetic parameters such as bioavailability and underlying parameters such as clearance rate may also affect efficacy. Thus, "enhanced efficacy" (for example, improvement in efficacy) may be due to enhanced pharmacokinetic parameters and improved efficacy, which may be measured by comparing parameters such as clearance rate and treatment or amelioration of a metabolic disease in test animals or human subjects.

Meanwhile, the pharmaceutical composition according to the present invention is administered in a "therapeutically effective amount".

As used herein, the term "administration" refers to introducing a predetermined substance to a subject by an appropriate method, and the route of administration of the composition may be through any general route as long as it may reach a target tissue. They may be intraperitoneal administration, intravenous administration, intramuscular administration, subcutaneous administration, intradermal administration, topical administration, intranasal administration, intrapulmonary administration, or intrarectal administration, but are not limited thereto.

As used herein, the term "therapeutically effective amount" or "pharmaceutically effective amount" refers to an amount of a compound or composition effective to prevent or treat the disease in question, which is sufficient to treat the disease at a reasonable benefit/risk ratio applicable to medical treatment and does not cause adverse effects. A level of the effective amount may be determined depending on factors including the patient's health condition, type and severity of disease, activity of drug, the patient's sensitivity to drug, mode of administration, time of administration, route of administration and excretion rate, duration of treatment, formulation or simultaneously used drugs, and other factors well known in the medical field. In an embodiment, the therapeutically effective amount means an amount of a drug effective to treat a metabolic disease.

Here, the pharmaceutical composition may further comprise a pharmaceutically acceptable carrier. The pharmaceutically acceptable carrier may be any carrier as long as the carrier is a non-toxic substance suitable for delivery to a patient. Distilled water, alcohol, fat, wax, and inert solid may be contained as the carrier. A pharmaceutically acceptable adjuvant (buffer, dispersant) may also be contained in the pharmaceutical composition.

Specifically, by including a pharmaceutically acceptable carrier, the pharmaceutical composition may be prepared into a parenteral formulation depending on its route of administration using conventional methods known in the art. Here, the term "pharmaceutically acceptable" means that the carrier does not have more toxicity than the subject to be applied (prescribed) can adapt while not inhibiting activity of the active ingredient.

When the pharmaceutical composition is prepared into a parenteral formulation, it may be made into preparations in the form of injections, transdermal patches, nasal inhalants, or suppositories with suitable carriers according to methods known in the art. In a case of being made into injections, sterile water, ethanol, polyol such as glycerol or propylene glycol, or a mixture thereof may be used as a suitable carrier; and an isotonic solution, such as Ringer's solution, phosphate buffered saline (PBS) containing triethanol amine or sterile water for injection, and 5% dextrose, or the like may preferably be used. Formulation of pharmaceutical compositions is known in the art, and reference may specifically be made to Remington's Pharmaceutical Sciences (19th ed., 1995) and the like. This document is considered part of the present description.

A preferred dose of the pharmaceutical composition may range from 0.01 *µ*g/kg to 10 g/kg, or 0.01 mg/kg to 1 g/kg, per day, depending on the patient's condition, body weight, sex, age, severity of the patient, and route of administration. The dose may be administered once a day or may be divided into several times a day. Such a dose should not be construed as limiting the scope of the present invention in any aspect.

Subjects to which the pharmaceutical composition can be applied (prescribed) are mammals and humans, with humans being particularly preferred. The pharmaceutical composition according to the present invention may further contain any compound or natural extract that is known to have a therapeutic effect on a metabolic disease.

In yet still another aspect of the present invention, there is provided a use of the fusion protein dimer for the manufacture of a medicament for treatment or prevention of a metabolic disease.

In yet still another aspect of the present invention, there is provided a use of the fusion protein dimer for treatment or prevention of a metabolic disease. Here, the fusion protein dimer, the metabolic disease, the prevention, and the treatment are as described above.

In yet still another aspect of the present invention, there is provided a method for preventing or treating a metabolic disease, the method including administering the fusion protein dimer to a subject. Here, the fusion protein dimer, the administration, the metabolic disease, the prevention, and the treatment are as described above. The subject may be a mammal, with a human being preferred. In addition, the subject may be a patient suffering from a metabolic disease, or an individual who is more likely to suffer from a metabolic disease.

Route of administration, dose, and frequency of administration of the fusion protein dimer may vary depending on the patient's condition and the presence or absence of side effects, and thus the fusion protein dimer may be administered to a subject in various ways and amounts, while the optimal administration method, dose, and frequency of administration can be selected in an appropriate range by those skilled in the art. In addition, the fusion protein dimer may be administered in combination with any compound or natural extract that is known to have a therapeutic effect on a metabolic disease or may be formulated in the form of combination preparations with other drugs.

### Mode for Carrying out the Invention

Hereinafter, the present invention will be described in more detail by way of the following examples. These examples are only for illustrating the present invention, and it will be apparent to one of ordinary skill in the art that the scope of the present invention is not to be construed as being limited by these examples.

### Preparation Example 1. Preparation of GLP-1-IgG4 Fc-IL-1Ra fusion protein dimer: GI-210B1

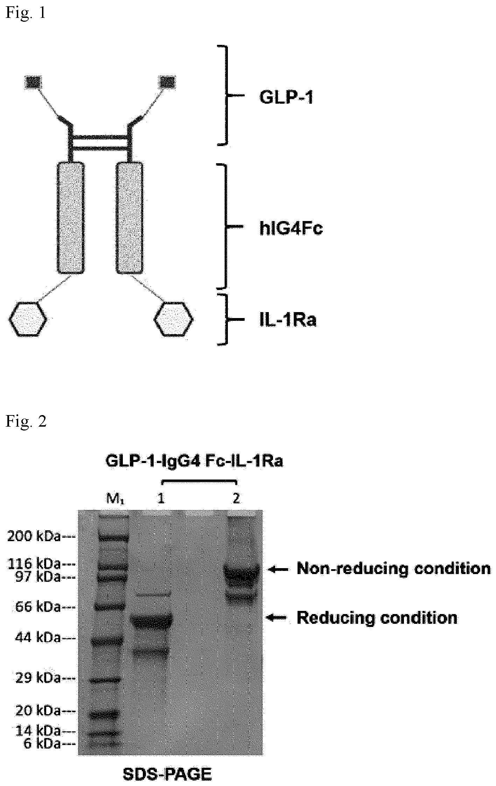

In order to produce a fusion protein dimer comprising glucagon-like peptide-1 (GLP-1), an Fc domain, and an interleukin-1 receptor antagonist (IL-1 receptor antagonist, II,-1Ra), a polynucleotide (SEQ ID NO: 20) encoding a fusion protein (FIG. 1) comprising GLP-1 (SEQ ID NO: 2), an NL028 hinge linker (SEQ ID NO: 5), an IgG4 Fc domain (SEQ ID NO: 6), a linker (SEQ ID NO: 7), and an II,-1Ra (SEQ ID NO: 8) was inserted into pcDNA3.4 vector (Genscript). The vector was introduced into CHO cells (ExpiCHO-S cells) to express a fusion protein having the amino acid sequence of SEQ ID NO: 10. After the vector was introduced, culture was performed for 14 days in a serum-free ExpiCHO^{™} expression medium (Thermo Fisher Scientific, Inc.) of 37°C and 8% CO₂ concentration. Then, the culture was harvested, and the fusion protein was purified therefrom using affinity chromatography (affinity purification column).

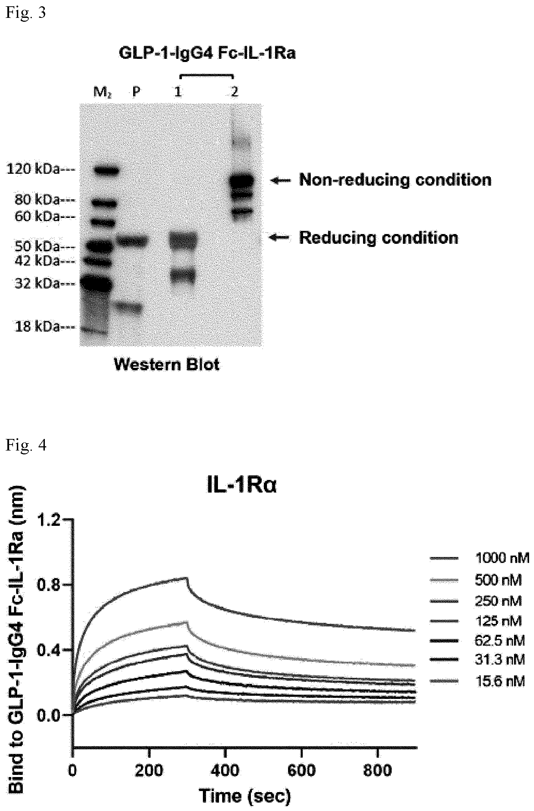

The purified fusion protein was subjected to SDS-PAGE and western blot to confirm the molecular weight and purity thereof. As a result of the SDS-PAGE and western blot analysis, it was confirmed that the protein was detected under non-reducing condition and reducing condition. Through this, it was found that the purified fusion protein formed a dimer (FIGS. 2 and 3). The fusion protein dimer was designated "GI-210B1 (also referred to as GLP-1-IgG4 Fc-II,-1Ra)".

### Preparation Example 2. Preparation of GLP-1-IgG4 Fc fusion protein dimer

In order to produce, as a control group, a fusion protein dimer comprising glucagon-like peptide-1 (GLP-1) and an Fc domain, a polynucleotide (SEQ ID NO: 23) encoding a fusion protein comprising GLP-1 (SEQ ID NO: 2), an NL028 hinge linker (SEQ ID NO: 5), and an IgG4 Fc domain (SEQ ID NO: 6) was inserted into pcDNA3.4 vector (Genscript). The vector was introduced into CHO cells (ExpiCHO-S cells) to express a fusion protein having the amino acid sequence of SEQ ID NO: 22. After the vector was introduced, culture was performed for 14 days in a serum-free ExpiCHO^{™} expression medium (Thermo Fisher Scientific, Inc.) 37°C and 8% CO₂ concentration. Then, the culture was harvested, and the fusion protein was purified therefrom using affinity chromatography (affinity purification column).

### Experimental Example 1. Identification of binding capacity of GI-210B1 fusion protein dimer to IL-1Rα

The binding capacity of GI-210B1, obtained by the method of Preparation Example 1, to interleukin-1 receptor alpha (IL-1Rα) was measured. Here, the binding capacity to IL-1Rα was checked using Octet RED384 (ForteBio). An amine reactive 2^{nd} generation (AR2G) biosensor was immersed in a 1X kinetic buffer for 10 minutes, and then used. IL-1Rα (R&D systems) was prepared at 1 µg/ml, the biosensor was coated with the IL-1Rα, the GI-210B1 was serially diluted to 15.6 to 1,000 nM, and then the binding capacity was measured at each concentration. The binding ability between GI-210B1 and IL-1Rα is as shown in FIG. 4 and Table 2 below.

**[Table 2]**

| Kₐ (Association rate, 1/Ms) | K_{d} (Dissociation rate, 1/s) | K_{D} (K_{d}/Kₐ, M) |
|---|---|---|
| 2.20E+04 | 9.30E-03 | 423 nM |

### Experimental Example 2. Identification of IL-1Ra efficacy of GI-210B1 fusion protein dimer

The efficacy of GI-210B1 as an IL-1 receptor antagonist (IL-1Ra) was confirmed. On the first day, THP-1 cells (ATCC) were cultured in a culture medium (RPMI-1640 + 10% FBS + 1% penicillin/streptomycin + 0.05 nM 2-mercaptoethanol). Prior to analysis, the THP-1 cells were precipitated through centrifugation at 1,500 rpm for 5 minutes, the medium was removed, and then the cells were resuspended. 3 × 10⁵ cells were inoculated into each well of a 96-well plate, then LPS (1 µg/ml) was added thereto, and culture was performed for 3 hours. Thereafter, the cells were treated with 5 mM of adenosine triphosphate (ATP) as an inducer of inflammasomes, which are a mediator of inflammatory responses, and cultured overnight. A supernatant was subjected to precipitation through centrifugation at 1,500 rpm for 5 minutes, and the resulting supernatant was transferred to a new tube and stored.

On the second day, HEK-Blue^{™}-IL-1β cells (Invitrogen) were cultured in a culture medium (DMEM + 10% FBS + 1% penicillin/streptomycin + 100 µg/ml normocin). Prior to analysis, the HEK-Blue^{™}-IL-1β cells were precipitated through centrifugation at 1,500 rpm for 5 minutes, the medium was removed, and then the cells were resuspended in an assay medium (RPMI-1640 + 10% FBS + 1% penicillin/streptomycin). 4.5 × 10⁴ cells in the assay medium were inoculated into each well of a 96-well plate, then the GI-210B 1 fusion protein at various concentrations (0.2 ng/ml, 2 ng/ml, 20 ng/ml, and 200 ng/ml) and 50 µL of the THP-1 supernatant were added thereto, and culture was performed overnight.

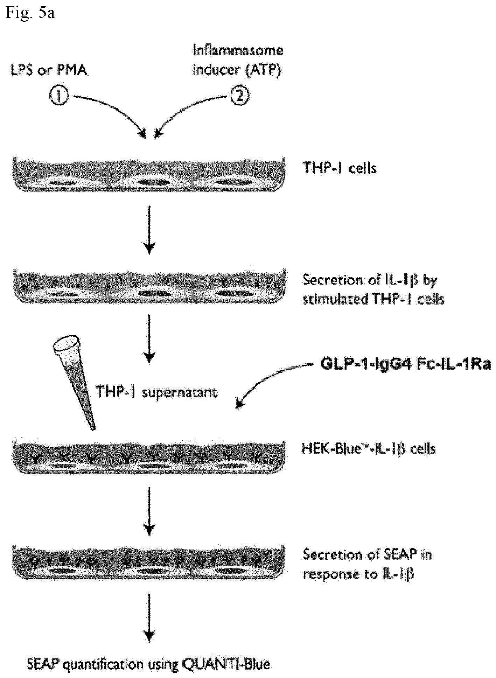

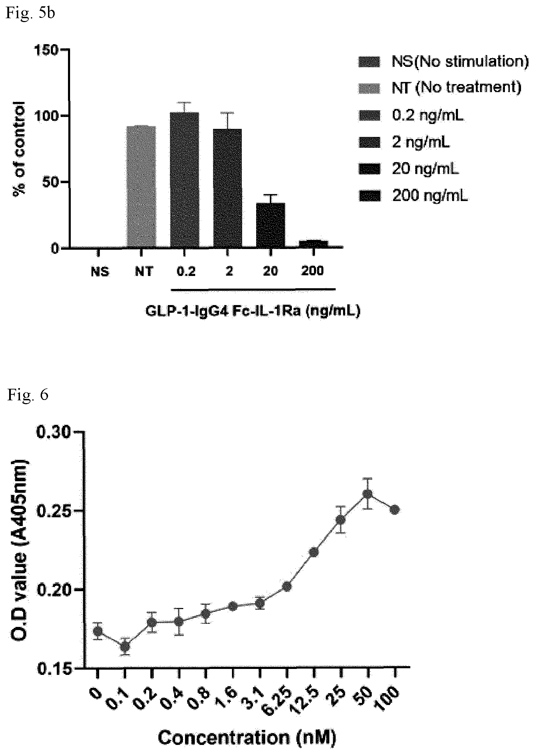

On the third day, a supernatant was subjected to precipitation through centrifugation at 1,500 rpm for 5 minutes, and 20 µL of the resulting supernatant and 180 µl of a QUANTI-Blue solution were dispensed into each well of a 96-well plate, followed by a reaction for 3 hours. Thereafter, the absorbance at a wavelength of 650 nm was recorded using a plate reader. The data were analyzed using GraphPad Prism software. As a result, it was confirmed that the GI-210B1 fusion protein inhibited the IL-1 signaling in a concentration-dependent manner (FIGS. 5a and 5b).

### Experimental Example 3. Identification of GLP-1 efficacy of GI-210B1 fusion protein dimer

In order to confirm the efficacy of GLP-1 contained in GI-210B1, an experiment was performed using PC-12 cells, which are a cell line dependent on GLP-1 in cell growth. That is, cell proliferation mediated by GLP-1 contained in the GI-210B 1 fusion protein was measured in the PC-12 cells.

First, PC-12 cells (ATCC, #CRL-1721^{™}) were cultured in a culture medium (RPMI-1640 + 10% FBS + 1% penicillin/streptomycin). Prior to analysis, the PC-12 cells were precipitated through centrifugation at 1,500 rpm for 5 minutes, the medium was removed, and then the cells were resuspended in an FBS-free assay medium. 5 × 10⁴ cells in the assay medium were inoculated into each well of a 96-well plate. The GI-210B 1 fusion protein at various concentrations (0.1 nM, 0.2 nM, 0.4 nM, 0.8 nM, 1.6 nM, 3.1 nM, 6.25 nM, 12.5 nM, 25 nM, 50 nM, and 100 nM) was added to the wells. The assay plate was cultured at 5% CO₂ and 37°C for 24 hours. Thereafter, 10 µl of WST-1 (Roche) was added to each well and cultured for 4 hours. Then, the absorbance at a wavelength of 450 nm was recorded using a plate reader. The data were analyzed using GraphPad Prism software. As a result, it was found that the GI-210B1 fusion protein increased the growth of the PC-12 cells in a concentration-dependent manner (FIG. 6).

### Experimental Example 4. Identification of ability of GI-210B1 fusion protein dimer to inhibit fat accumulation in hepatocytes caused by palmitate and oleic acid

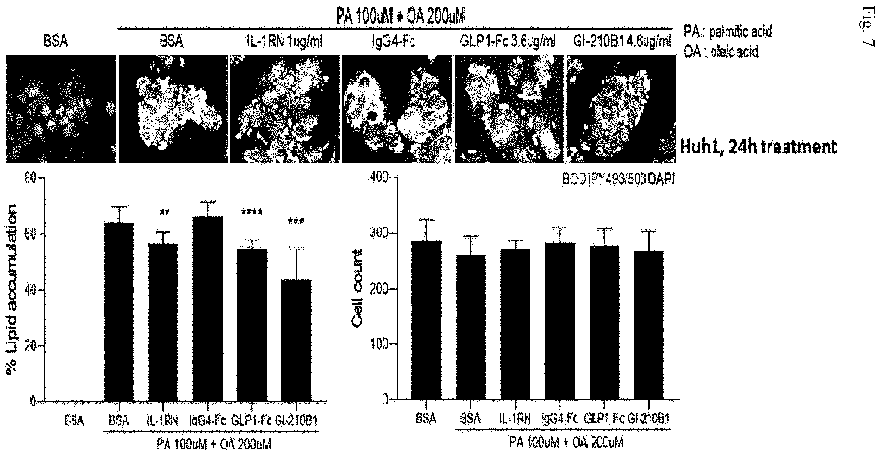

In order to confirm whether GI-210B1 inhibits IL-1β-induced fat accumulation in hepatocytes, an experiment was performed using Huh1 cells as hepatocytes. The Huh1 cells were cultured in an incubator at 5% CO₂ and 37°C using a culture medium (DMEM + 10% FBS + 1% penicillin/streptomycin). For the experiment, the cells were detached using 0.05% Trypsin-EDTA, and then precipitated through centrifugation at 1,500 rpm for 5 minutes. Thereafter, the cells were resuspended in a fresh medium, 1 × 10⁴ cells were inoculated into each well of a 96-well plate, and culture was performed for 24 hours. In order to induce fat accumulation, 100 µM palmitate and 200 µM oleic acid were mixed in 2% BSA-serum free DMEM, and reacted at 55°C for 15 minutes, and when treated therewith, the cells were also treated with 1 µg/ml IL-1Ra (peprotech, 200-01RA), 3.6 µg/ml GLP-1-Fc, or 4.6 µg/ml GI-210B 1, respectively. After 24 hours of the treatment, in order to confirm the degree of fat accumulation, the cells were immobilized with 4% paraformaldehyde and stained with BODIPY 493/503 and DAPI, and then the degree of fat accumulation per cell was quantified using cytation 5 imaging equipment. As a result, it was confirmed that the GI-210B1 fusion protein significantly inhibited the fat accumulation induced by palmitate and oleic acid, compared to IL-1Ra or GLP-1-Fc (FIG. 7).

## Claims

1. A fusion protein comprising:
GLP-1 or a variant thereof; and
an interleukin-1 receptor antagonist (IL-1 receptor antagonist) or a fragment thereof.

2. The fusion protein of claim 1, wherein the fusion protein comprises an immunoglobulin Fc region.

3. The fusion protein of claim 1, wherein the GLP-1 has the amino acid sequence of SEQ ID NO: 1.

4. The fusion protein of claim 1, wherein the variant of GLP-1 has the amino acid sequence of any one of SEQ ID NOs: 2 to 4.

5. The fusion protein of claim 1, wherein the IL-1 receptor antagonist has the amino acid sequence of SEQ ID NO: 8.

6. The fusion protein of claim 1, wherein the fusion protein consists of the following structural formula (I) or (II):
N'-X-[linker (1)]n-Fc region fragment or variant thereof-[linker (2)]m-Y-C' (I)
N'-Y-[linker (1)]n-Fc region fragment or variant thereof-[linker (2)]m-X-C' (II) in the structural formulae (I) and (II),
N' is the N-terminus of the fusion protein,
C' is the C-terminus of the fusion protein,
X is GLP-1 or a variant thereof,
Y is an IL-1 receptor antagonist or a fragment thereof,
the linker (1) and the linker (2) are peptide linkers, and
n and m are each independently 0 or 1.

7. The fusion protein of claim 6, wherein the Fc region of the fusion protein is derived from human IgG4.

8. A fusion protein dimer in which two fusion proteins of any one of claims 1 to 7 are bound.

9. A polynucleotide encoding the fusion protein of any one of claims 1 to 7.

10. An expression vector comprising the polynucleotide of claim 9.

11. A transformed cell into which the vector of claim 10 has been introduced.

12. A method for preparing a fusion protein dimer which comprises GLP-1 or a variant thereof and an interleukin-1 receptor antagonist (IL-1 receptor antagonist) or a fragment thereof, the method including:
(i) culturing the transformed cell of claim 11; and
(ii) collecting a fusion protein dimer.

13. A pharmaceutical composition for preventing or treating a metabolic disease, comprising the fusion protein dimer of claim 8.

14. The pharmaceutical composition of claim 13, wherein the metabolic disease is any one selected from the group consisting of diabetes, obesity, hyperlipidemia, hypertension, arteriosclerosis, and hyperinsulinemia.

15. Use of the fusion protein dimer of claim 8 for the manufacture of a medicament for treatment or prevention of a metabolic disease.

16. Use of the fusion protein dimer of claim 8 for treatment or prevention of a metabolic disease.

17. A method for preventing or treating a metabolic disease, comprising administering the fusion protein dimer of claim 8 to a subject.
